# FASCICULE DE BREVET EUROPEEN

(11) **EP 4 385 411 B1**
(45) Date de publication et mention de la délivrance du brevet: **15.04.2026**
(21) Numéro de dépôt: 24173679.2
(22) Date de dépôt: 12.02.2019
(51) Int. Cl.: A61B 5/291

(54) **ELECTROENCEPHALOGRAPHES PORTATIFS**
TRAGBARE ELEKTROENZEPHALOGRAPHEN
PORTABLE ELECTROENCEPHALOGRAPHS

(30) Priorité: 15.02.2018 FR 1851287
(43) Date de publication de la demande: 19.06.2024
(62) Demande divisionnaire de: 19708238.1
(73) Titulaire: Specs France SAS, 75008 Paris (FR)
(72) Inventeur: KOUIDER, Sid, 75001 Paris (FR); ZHANG, Hao, 92150 Paris (FR); PLOYART, Guillaume, 75010 Paris (FR); GOUPILLE, Antoine, 75019 Paris (FR); VICERIAL, Jeanne, 93500 Paris (FR); BIANCARELLI, Arthur, 75017 Paris (FR); GERVAIS, Gaelle, 75011 Paris (FR)
(74) Mandataire: Mathys & Squire

(56) Documents cités:
- WO-A1-2016/140325
- WO-A1-2016/166740
- CN-A- 107 049 307
- JP-B1- 3 626 176
- US-A1- 2015 112 153
- US-A1- 2016 022 981
- US-A1- 2016 157 777
- US-A1- 2017 258 400

## Description

### ETAT DE L'ART

### Domaine technique de l'invention

La présente invention concerne des dispositifs portatifs pour l'acquisition de signaux électroencéphalographiques (EEG) ou électroencéphalographes portatifs de surface, ainsi que des méthodes d'acquisition de signaux électroencéphalographiques utilisant ces électro encéphalo graphes.

### Etat de l'art

L'électroencéphalographie de surface permet de mesurer les variations de potentiels électriques diffus à la surface du crâne. Ces variations de potentiels électriques sont couramment appelés signaux électroencéphalographiques ou signaux EEG.

Une première difficulté concerne la fiabilité des dispositifs permettant de capturer les signaux EEG. En effet, compte tenu de la très faible amplitude des variations de potentiels électriques à mesurer (de l'ordre de quelques microvolts), il est nécessaire d'assurer une conductivité maximale entre l'électrode et le scalp afin d'obtenir un signal EEG exploitable, et donc un parfait contact, ce qui peut s'avérer difficile du fait notamment de la chevelure de l'utilisateur.

Plusieurs solutions techniques sont aujourd'hui utilisées dans les dispositifs actuels pour répondre à la contrainte de fiabilité des signaux.

Certains électroencéphalographes de surface sont équipés d'électrodes à gel, dans lesquelles le contact s'effectue par le biais d'un gel ou d'un liquide conducteur, qui s'infiltre aisément à travers la chevelure de l'utilisateur pour atteindre le scalp. Le gel permet de réduire l'impédance électrique et ainsi les interférences avec des signaux environnants. Cette solution permet d'obtenir une bonne conductivité en tout point du scalp. Cependant, ce type de dispositif nécessite une assistance technique pour la pose des électrodes. En particulier, cette solution est chronophage (le gel est placé et la conductance vérifiée sur chaque électrode une par une). De plus, elle limite la durée d'utilisation du dispositif à quelques heures (le gel séchant, le contact n'est plus assuré).

Plus récemment, on a vu se développer des électroencéphalographes de surface équipés d'électrodes sèches dites « actives ». De telles électrodes sont par exemple décrites dans la demande de brevet publiée US 20133066183. Les électrodes sèches actives ont pour fonction de capter les variations de potentiels électriques à la surface du cuir chevelu, de les filtrer et de les amplifier. Les signaux analogiques ainsi obtenus sont ensuite convertis en signaux numériques au moyen d'un ou plusieurs convertisseurs analogique-numérique commandés par un microcontrôleur. Celui-ci reçoit les données pour les analyser, pour les stocker ou pour les transmettre vers un autre dispositif.

Dans les électrodes sèches actives, le contact avec le cuir chevelu se fait par le biais d'éléments conducteurs solides ou « capteurs » reliés à un circuit électronique permettant de pallier l'augmentation de l'impédance due à l'absence de gel. Le composant électronique actif permet d'assurer une capture des signaux comparable à celle d'une électrode à gel. Un avantage supplémentaire de l'électrode active est qu'elle permet de filtrer et/ou d'amplifier les signaux, et ainsi d'améliorer le rapport signal sur bruit. Cependant, la principale difficulté de cette technique est l'accès au cuir chevelu.

Les solutions actuelles utilisent généralement des capteurs à base de polymère et à picots en exerçant une pression importante pour atteindre le scalp de l'utilisateur (voir par exemple la demande de brevet publiée US 2015141788. Si cette approche peut permettre un bon contact avec le scalp, elle a l'inconvénient majeur d'être très inconfortable, surtout pour une utilisation prolongée.

Par ailleurs, une autre difficulté dans la conception des électroencéphalographes de surface concerne l'acceptabilité de l'EEG auprès du grand public, qui impose des contraintes esthétiques, de confort et de facilité d'utilisation.

En effet, les systèmes à destination du domaine médical ou de la recherche comprennent généralement un bonnet, en tissu élastique ou imperméable, avec des emplacements destinés à accueillir les capteurs, les circuits électroniques reliés aux capteurs et le boîtier de la chaîne d'acquisition. Ils sont ainsi formés de trois éléments distincts que l'opérateur/appariteur doit assembler à chaque utilisation.

Des dispositifs portatifs pour l'acquisition de signaux EEG ont été proposés qui permettent à un utilisateur de s'affranchir d'une assistance d'un technicien spécialisé. La demande de brevet US 2002/0029005 par exemple, décrit un couvre-chef pour l'acquisition de signaux EEG avec des emplacements prédéterminés pour les électrodes et des bandes élastiques réglables permettant d'assurer le contact des électrodes contre le cuir chevelu. Un tel dispositif reste cependant complexe dans son utilisation du fait du grand nombre de pièces distinctes et réglables.

La demande de brevet US 2017/0027466 décrit également un dispositif portatif pour l'acquisition de signaux EEG utilisable sans assistance, et dans lequel le nombre de pièces mécaniques amovibles et réglables est réduit, permettant une utilisation plus simple et rapide pour un utilisateur non expérimenté. Pour ce faire, le dispositif d'acquisition de signaux EEG comprend une pièce centrale destinée à être positionnée sur le haut de la tête et pouvant abriter l'ensemble des composants électroniques. De la pièce centrale s'étendent tout autour de la tête des bras longs et courts au bout desquels sont positionnées des capteurs. Certains de ces bras au moins sont élastiques ou présentent des fonctions ressort qui permettent la préhension de la tête en s'adaptant à son contour, avec une force suffisante pour assurer le contact nécessaire des capteurs avec le cuir chevelu. Outre le manque de discrétion d'un tel dispositif, celui-ci présente l'inconvénient d'être très inconfortable puisque, sauf à maintenir une position tête droite à la verticale, la force de préhension passe exclusivement par les capteurs, ce qui et génère des points de forte pression très localisés.

La demande de brevet US 2016/157777 décrit également un dispositif portatif pour l'acquisition de signaux EEG comprenant une pièce centrale destinée à être positionnée sur le haut de la tête, et de laquelle s'étendent des bras longs et courts au bout desquels sont positionnés des capteurs. L'ensemble des composants électroniques peuvent être abrités au sein de la pièce centrale ou de l'une des branches flexibles.

Les demandes de brevet US 2015/112453 et US 2017/258400 décrivent des dispositifs portatifs pour l'acquisition de signaux EEG dans lesquelles au moins une partie des composants électroniques peuvent être logés dans des assemblages pouvant se connecter à un connecteur électronique du dispositif. Dans le dispositif décrit dans la demande de brevet US 2015/112453, l'assemblage peut être connecté de façon détachable.

Les dispositifs décrits, même s'ils permettent de s'affranchir du support technique d'un opérateur et proposent des solutions permettant d'assurer un contact satisfaisant avec le cuir chevelu de l'utilisateur, manquent de la discrétion nécessaire notamment pour des applications grand public, comme par exemple les jeux vidéo, la formation, l'aide au sommeil etc.

La présente description propose des électroencéphalographes portatifs de surface équipés d'électrodes sèches actives qui offrent en plus d'une excellente qualité du signal, une grande facilité d'utilisation, un confort pour l'utilisateur et une grande discrétion. De tels électroencéphalographes portatifs de surface pourront être utilisés en milieu hospitalier, par exemple en ambulatoire dans un contexte de diagnostic clinique, mais permettent également de favoriser l'émergence de nouveaux domaines d'applications pour l'électroencéphalographie.
US 2016/022981 divulgue un système à electrodes pour stimulation électrique.

### RESUME

L'invention est définie par les revendications annexées.

Selon un premier aspect, la présente description décrit aussi un dispositif portatif pour l'acquisition de signaux électroencéphalographiques (EEG) émis par un utilisateur, le dispositif comprenant :
- un support flexible destiné à épouser une région localisée du crâne de l'utilisateur;
- un ensemble de capteurs pour la détection de signaux électriques générés par l'activité neuronale de l'utilisateur, agencés sur ledit support de telle sorte à former des contacts avec le cuir chevelu lorsque le dispositif est porté par l'utilisateur ;
- pour chaque capteur, un circuit électronique de filtrage et d'amplification des signaux électriques détectés par ledit capteur, ledit circuit électronique étant intégré dans le support flexible et formant avec ledit capteur une électrode active;
- un boitier comprenant une chaîne électronique de traitement des signaux issus desdits circuits électroniques de filtrage et d'amplification, ledit boitier étant relié mécaniquement au support flexible pour former, avec ledit support, un moyen d'attache à un vêtement ou accessoire destiné à être porté par l'utilisateur.

Dans le dispositif ainsi décrit, les composants électroniques assurant les différentes fonctions électroniques sont répartis entre le support flexible et le boitier, ces derniers coopérant mécaniquement pour former un moyen d'attache à un vêtement ou accessoire. Cela permet à la fois de rendre le support destiné à être en contact avec le crâne de l'utilisateur plus flexible et plus fin, typiquement d'épaisseur inférieure à 10 mm, voire d'épaisseur inférieure 5 mm, et de rendre le dispositif extrêmement facile d'utilisation, sans perdre en qualité des signaux EEG acquis. Le dispositif portatif ainsi décrit présente donc des performances au moins comparables à celles des dispositifs de l'état de l'art, mais aussi une facilité d'utilisation et une ergonomie compatible avec les applications grand public.

Le dispositif portatif s'applique à l'homme mais peut également être appliqué à certains animaux, le dispositif, non invasif, présentant des qualités de précision et de confort propices à une utilisation en recherche sur les animaux.

Le nombre de capteurs agencés sur le support flexible dépend de l'application. Ce nombre peut être par exemple compris entre 2 et 128, voire même davantage. Selon l'application envisagée, le support peut soit couvrir une zone limitée du crâne pour mesurer l'activité cérébrale générée par une zone précise du cerveau, par exemple le cortex visuel, auditif, moteur, somatosensoriel, ou préfrontal, soit s'étendre sur toute la surface du crâne. Le nombre de capteurs pourra être déterminé en fonction de la surface couverte par le support et de la résolution spatiale voulue.

Selon un ou plusieurs exemples de réalisation, le support flexible et le boitier sont reliés mécaniquement par un point d'attache, par exemple un point d'attache décentré pour former, entre le boitier et le support, un interstice permettant de laisser passer un vêtement et/ou un accessoire. Avantageusement, ledit interstice est compris entre 2 mm et 5 mm.

Selon un ou plusieurs exemples de réalisation, le point d'attache est flexible permettant de former un moyen d'attache en forme de « clip » ou pince.

Selon un ou plusieurs exemples de réalisation, le point d'attache permet en outre le contact électrique entre les composants électroniques logés dans le support flexible et ceux logés dans le boîtier.

Selon un ou plusieurs exemples de réalisation, le point d'attache est détachable.

Selon un ou plusieurs exemples de réalisation, la chaîne électronique de traitement des signaux logée dans le boitier rigide comprend un ou plusieurs convertisseurs analogique/numérique (CAN) destiné à transformer les signaux issus des circuits électroniques de filtrage et d'amplification en signaux numériques et un microcontrôleur, notamment pour la transmission vers une unité de traitement externe et/ou le stockage desdits signaux numériques. Le boîtier rigide peut bien entendu loger d'autres éléments électroniques, par exemple une batterie, et/ou d'autres types de capteurs, par exemple un accéléromètre et/ou un gyroscope.

Selon un ou plusieurs exemples de réalisation, le support flexible est ajouré ; par exemple, le support flexible comprend une pluralité de branches sur lesquelles sont agencés au moins une partie desdits capteurs. Cette structure confère une plus grande flexibilité au support et permet une meilleure adaptation à la forme du crâne.

Par exemple, les capteurs sont répartis sur 2 à 6 branches.

Selon un ou plusieurs exemples de réalisation, les branches sont parallèles, ce qui permet d'appliquer une pression plus uniforme sur les capteurs même lorsque le vêtement ou ustensile ne recouvre pas l'ensemble du support, et facilite la compréhension du geste de translation qu'un utilisateur pourra faire pour mettre en place le dispositif.

Selon un ou plusieurs exemples de réalisation, 2 branches ou davantage peuvent être reliées à une partie centrale, au moyen de branches latérales flexibles.

Selon un ou plusieurs exemples de réalisation, 3 branches ou davantage peuvent être agencées parallèlement en forme de peigne.

Selon un ou plusieurs exemples de réalisation, chaque capteur est monté mobile sur ledit support flexible, par exemple au moyen d'une liaison mécanique de type ressort, ce qui permet d'améliorer le contact avec le cuir chevelu.

Selon un ou plusieurs exemples de réalisation, la liaison mécanique de type ressort comprend une lame ressort formant un contact ponctuel avec une base dudit capteur, permettant une mobilité du capteur selon plusieurs axes.

Selon un ou plusieurs exemples de réalisation, ladite lame ressort assure le contact électrique dudit capteur avec ledit circuit de filtrage et d'amplification.

Selon un ou plusieurs exemples de réalisation, chaque capteur comprend une base destinée à être agencée dans un logement du support flexible, en contact électrique avec ledit circuit de filtrage et d'amplification.

Selon un ou plusieurs exemples de réalisation, chaque capteur comprend une pluralité de lamelles conductrices, agencées sur ladite base, lesdites lamelles conductrices étant destinées à former des contacts linéiques avec le cuir chevelu lorsque le dispositif est porté par l'utilisateur.

Un tel contact linéique permet d'avoir une plus grande surface de contact et de ce fait une meilleure sensibilité et un meilleur confort pour l'utilisateur.

Selon un ou plusieurs exemples de réalisation, les lamelles conductrices sont agencées de façon sensiblement parallèle. Lorsqu'au moins une partie des capteurs sont agencés sur des branches parallèles du support, les lamelles conductrices sont avantageusement parallèles audites branches.

Selon un ou plusieurs exemples de réalisation, chaque capteur comprend deux lamelles conductrices. Le nombre de deux lamelles conductrices est un bon compromis car il permet de répartir la pression de contact tout en gardant une bonne précision de mesure.

Selon un ou plusieurs exemples de réalisation, l'espacement bord à bord entre lesdites deux lamelles conductrices est supérieur à 2 mm, pour pouvoir laisser passer les cheveux. Avantageusement, ledit espacement est inférieur à 50 mm, avantageusement inférieur à 10 mm pour ne pas perdre en précision. Par exemple, ledit espacement est compris entre 2 mm et 6 mm.

Selon un ou plusieurs exemples de réalisation, les lamelles conductrices comprennent un revêtement polymère conducteur formant une couche conductrice destinée à entrer en contact avec le scalp de l'utilisateur.

Selon un ou plusieurs exemples de réalisation, les lamelles conductrices présentent au moins une pointe (conductrice ou non) destinée à entrer en contact en premier avec le cuir chevelu lorsque le support est positionné sur le crâne. Cette pointe a pour effet d'écarter les cheveux lors de la mise en place du dispositif, de manière à exposer le scalp de l'utilisateur aux lamelles conductrices.

Selon un deuxième aspect, la présente description décrit aussi un vêtement ou accessoire connecté pour l'acquisition de signaux électroencéphalographiques (EEG) comprenant un dispositif portatif selon le premier aspect.

Ce vêtement ou accessoire peut être par exemple un bandeau, un couvre-chef, un casque, etc.

Selon un troisième aspect, la présente description décrit aussi un procédé d'acquisition de signaux électroencéphalographiques (EEG) émis par un utilisateur au moyen d'un dispositif portatif selon le premier aspect, comprenant :
- la mesure de signaux électriques générés par l'activité neuronale de l'utilisateur au moyen desdits capteurs en contact avec le cuir chevelu de l'utilisateur;
- le traitement des signaux électriques issus desdits circuits de filtrage et d'amplification au moyen de la chaîne électronique de traitement agencée dans ledit boitier.

Selon un ou plusieurs exemples de réalisation, ledit traitement des signaux électriques comprend la conversion analogique/numérique des signaux électriques issus desdits circuits de filtrage et d'amplification et la transmission desdits signaux numériques vers une unité de traitement externe et/ou le stockage desdits signaux numériques.

### BREVE DESCRIPTION DES FIGURES

D'autres avantages et caractéristiques de l'invention apparaîtront à la lecture de la description, illustrée par les figures suivantes qui représentent :
- FIGS. 1A - 1E, différentes vues d'un premier exemple d'un dispositif portatif pour l'acquisition de signaux EEG selon la présente description ;
- FIGS. 2A-2C, différentes vues d'un deuxième exemple d'un dispositif portatif pour l'acquisition de signaux EEG selon la présente description ;
- FIGS. 3A et 3B, différentes vues d'un troisième exemple d'un dispositif portatif pour l'acquisition de signaux EEG selon la présente description ;
- FIG. 4, un exemple d'architecture électronique pour la réception et le traitement des signaux EEG au moyen d'un dispositif portatif selon la présente description ;
- FIGS. 5A, 5B, des exemples de réalisation d'un capteur selon la présente description et FIG. 5C, un autre exemple de capteur selon la présente description ;
- FIG. 6, un exemple de réalisation d'un capteur monté mobile sur le support flexible.

### DESCRIPTION DETAILLEE

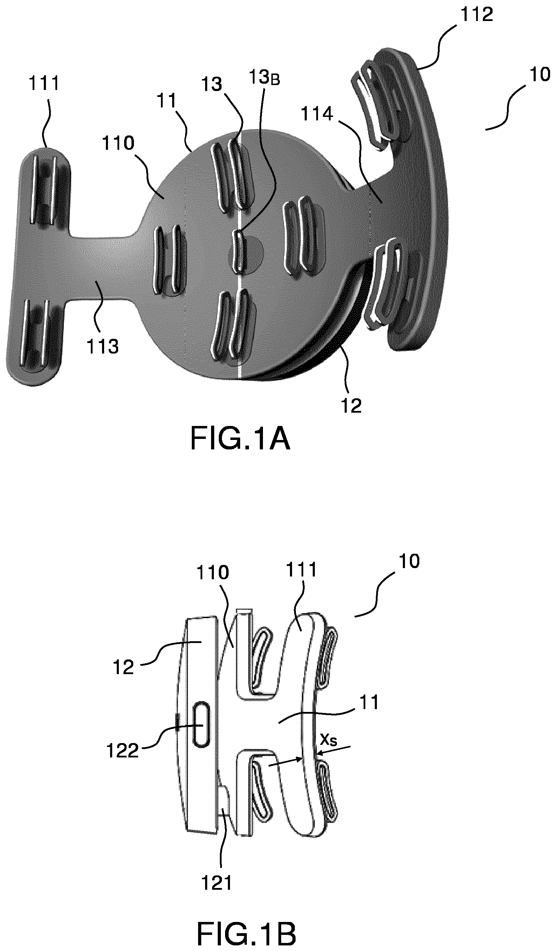

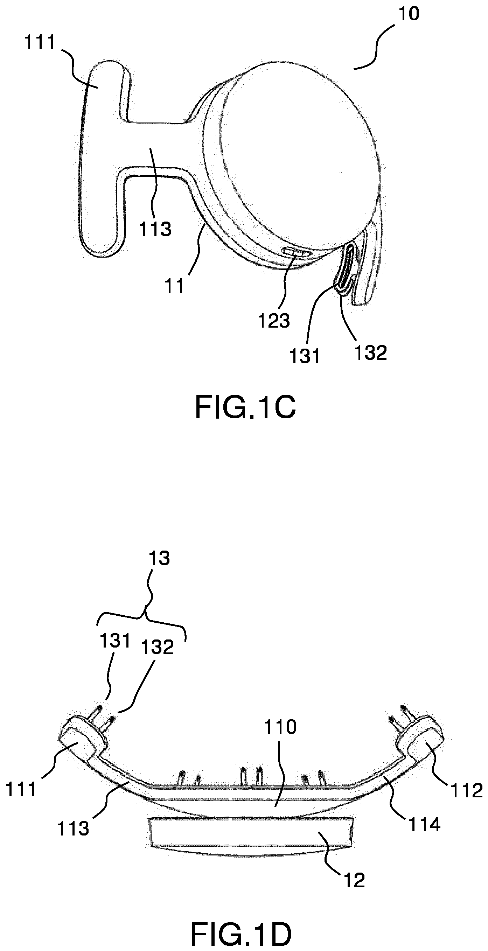

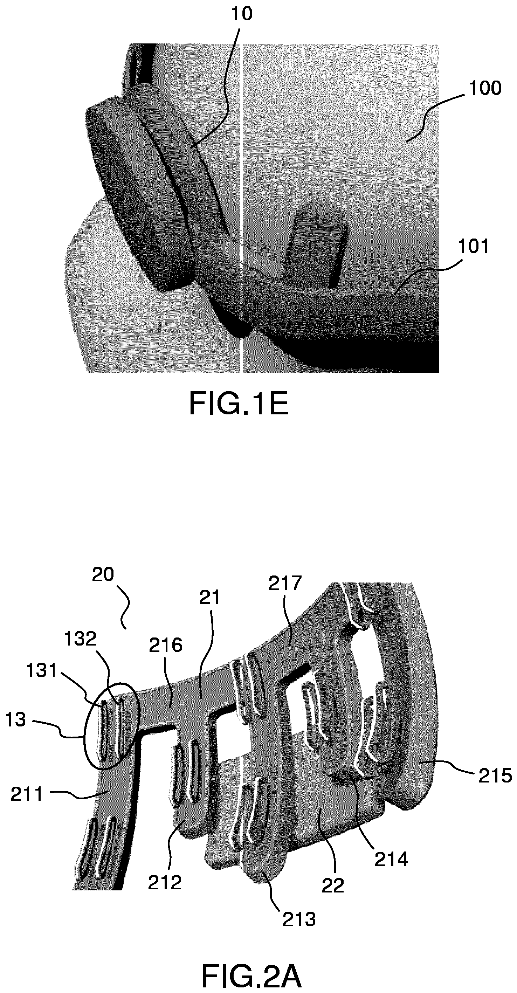

Les FIGS. 1A - 1D représentent respectivement une vue de face, une première vue de côté, une vue de dos et une deuxième vue de côté d'un premier exemple d'un dispositif portatif 10 pour l'acquisition de signaux EEG selon la présente description, tandis que la FIG. 1E illustre une vue du dispositif porté par un utilisateur.

Le dispositif portatif tel qu'illustré sur les FIGS. 1A à 1D comprend un support flexible 11 destiné à épouser une région localisée du crâne d'un utilisateur, par exemple la région occipitale à l'arrière du crâne, et un boîtier 12 relié mécaniquement et électroniquement au support flexible 11. Sur le support, sont agencés un ensemble de capteurs 13 pour la détection de signaux électriques générés par l'activité neuronale de l'utilisateur. Une électrode 13_{B} supplémentaire dite de mise à la masse ou « bias » est prévue pour supprimer le mode commun des signaux mesurés par les autres capteurs. Comme cela sera décrit plus en détails par la suite, chaque capteur peut comprendre une pluralité de lamelles conductrices (dans cet exemple deux lamelles conductrices 131, 132), par exemple agencées de façon sensiblement parallèle, de telle sorte à former des contacts linéiques avec le cuir chevelu lorsque le dispositif est porté par l'utilisateur ; ces lamelles conductrices peuvent en outre être déformables par pression sur le cuir chevelu de l'utilisateur. Chaque capteur forme avec un circuit électronique de filtrage et d'amplification des signaux électriques (non représenté sur les FIGS. 1A - 1D) une électrode active. Les signaux issus des circuits électroniques de filtrage et d'amplification sont traités par une chaîne électronique comprenant par exemple, comme cela sera décrit par la suite, un ou plusieurs convertisseurs analogique/numérique (CAN) et un microcontrôleur permettant notamment le stockage et/ou la transmission vers l'extérieur du dispositif des signaux traités. La chaine électronique est intégrée dans le boîtier 12 tandis que les circuits électroniques de filtrage et d'amplification des électrodes actives sont intégrés dans le support flexible. La séparation des composants électroniques respectivement dans le support sur lequel sont agencés les capteurs et dans le boîtier permet une plus grande souplesse de conception du support 11, notamment en termes de forme, de finesse et de flexibilité mécanique. Ainsi par exemple, l'épaisseur X_{S} du support flexible peut avantageusement être inférieure à 10 mm, par exemple comprise entre 2 mm et 10 mm, et peut être avantageusement inférieure à 5 mm, par exemple comprise entre 2 mm et 5 mm. Le boîtier peut bien entendu loger d'autres éléments électroniques nécessaires au fonctionnement du dispositif, par exemple une batterie et/ou d'autres types de capteurs, par exemple un accéléromètre et/ou un gyroscope. Dans l'exemple des FIGS 1A - 1D, le boitier 12 est par ailleurs équipé d'un interrupteur Marche/Arrêt 122 et d'un port de connexion 123, par exemple un port USB.

Par ailleurs, comme cela est illustré sur les FIGS 1A - 1E, le support 11 et le boîtier 12 coopèrent pour former un moyen d'attache à un vêtement ou accessoire destiné à être porté par l'utilisateur. Ainsi dans cet exemple, le boitier 12 est relié au support 11 par un point d'attache 121 décentré pour former, entre le boitier et le support, un interstice permettant de laisser passer un vêtement et/ou un accessoire, typiquement un interstice de quelques millimètres, par exemple entre 2 mm et 5 mm. Dans cet exemple, le point d'attache est flexible de telle sorte à former une pince ou « clip », dans laquelle on peut passer le vêtement ou un accessoire, par exemple un bandeau 101 comme illustré sur la FIG. 1E. Le bandeau élastique peut alors permettre de plaquer le support flexible contre le crâne 100 de l'utilisateur. Le point d'attache 121 forme également une liaison électrique entre les circuits de filtrage et d'amplification des électrodes actives et la chaine électronique du boitier. Par exemple, le point d'attache comprend un manchon à l'intérieur duquel peuvent passer des connexions électriques. Le point d'attache peut également être détachable.

Avantageusement, la forme du support est conçue de manière à garantir une répartition équitable de la pression entre les différents capteurs lorsque le dispositif est porté par l'utilisateur. Dans l'exemple des FIGS 1A - 1D, le support flexible 11 est ajouré, et comprend des branches extérieures 111, 112 reliées à une partie centrale 110 au moyen de branches latérales 113, 114 qui confèrent une flexibilité à l'ensemble. Les capteurs sont agencés sur les branches extérieures et la partie centrale. Dans cet exemple, la partie centrale 110 est sensiblement ronde et se superpose au boitier, la forme ronde du boitier facilitant la préhension avec la main.

Dans l'exemple des FIGS 1A à 1E, deux branches extérieures sont représentées, agencées latéralement pour, avec la partie centrale, permettre une répartition des capteurs sur la zone d'intérêt du crâne. En fonction des besoins, d'autres branches supportant des capteurs pourraient être prévues ; par exemple une troisième et quatrième branche pour le positionnement de capteurs sur les parties latérales du crâne.

Bien entendu, d'autres formes sont possibles pour le support flexible 11, notamment des formes ajourées.

Ainsi, les FIGS. 2A - 2C illustrent un deuxième exemple d'un dispositif portatif 20 pour l'acquisition de signaux EEG selon la présente description.

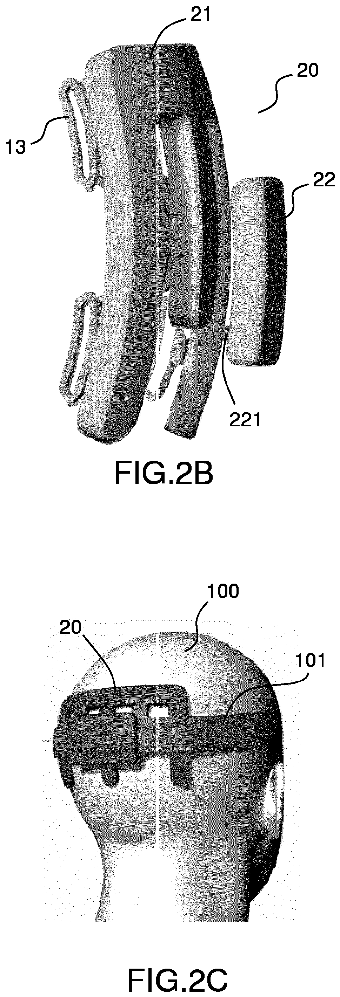

Les FIGS. 2A et 2B illustrent respectivement une vue de face et une vue de côté, tandis que la FIG. 2C montre le dispositif 20 porté par un utilisateur au moyen d'un bandeau 101. Le dispositif portatif comprend un support flexible 21 destiné à épouser une région localisée du crâne d'un utilisateur et un boîtier rigide 22 relié mécaniquement et électroniquement au support flexible 11. Le support flexible 21 est destiné à supporter, comme dans l'exemple précédent, des capteurs 13, chaque capteur formant, avec un circuit de filtrage et d'amplification (non représenté) une électrode active.

Dans cet exemple, le support 21 est également ajouré pour permettre une meilleure flexibilité. Il comprend dans cet exemple un nombre donné de branches 211 - 215 toutes agencées parallèlement et sur lesquelles sont agencés les capteurs 13. Bien qu'une électrode de mise à la masse ne soit pas représentée sur ces figures, elle peut bien sûr être prévue, comme dans l'exemple précédent. Dans cet exemple à nouveau, les branches 211 - 215 qui portent les capteurs sont reliées par des branches latérales 216 - 217 qui confèrent de la flexibilité à l'ensemble.

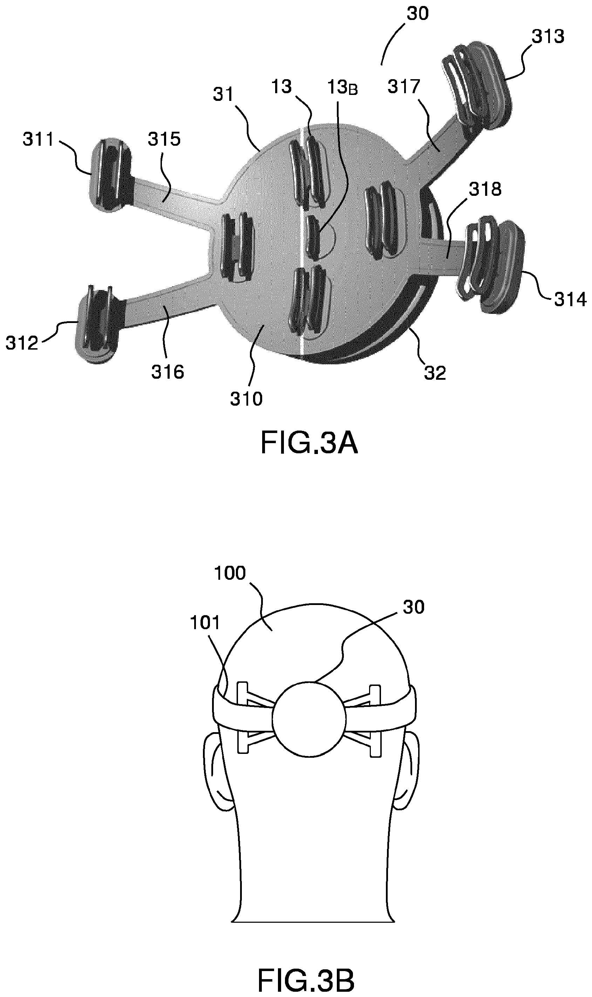

Dans cet exemple, le boîtier 22 et le support 21 auquel il est relié par un point d'attache 221, coopèrent pour former, comme dans l'exemple précédent, un moyen d'attache à un vêtement ou accessoire. Ainsi, comme illustré sur la FIG. 3C, un bandeau 101 peut être inséré dans l'interstice formé entre le support 21 et le boitier 22 pour maintenir le dispositif 20 contre le crâne 100 de l'utilisateur.

Les FIGS. 3A - 3B illustrent un troisième exemple d'un dispositif portatif 30 pour l'acquisition de signaux EEG selon la présente description.

Dans cet exemple à nouveau, le dispositif portatif 30 comprend un support flexible 31 et un boitier 32 reliés mécaniquement pour former un moyen d'attache à un vêtement ou ustensile. Par ailleurs, le support 31 et le boitier 32 sont connectés électriquement. Le support 31 est, dans cet exemple, ajouré de telle sorte à former une partie centrale 310 et un ensemble de branches extérieures 311 - 314 reliées à la partie centrale par des branches latérales 315 - 318 qui permettent de conférer de la flexibilité au support. Dans cet exemple comme dans celui des FIGS. 1A - 1E, la partie centrale 310 du support est sensiblement ronde et sa forme se superpose à celle du boitier 32 pour permettre une meilleure préhension par un utilisateur. Un ensemble de capteurs 13 sont agencés sur la partie centrale et sur les branches extérieures du support 31, ainsi qu'une électrode de mise à la masse 13_{B}.

Dans les exemples illustrés sur les figures précédentes, notamment les FIGS. 1E, 2C, 3B, le dispositif portatif (respectivement 10, 20 et 30) est appliqué contre le crâne de l'utilisateur au moyen d'un bandeau 101. On comprend aisément que le moyen d'attache formé par le boitier et le support peut être appliqué à tout autre type de vêtement ou d'accessoire, par exemple une casquette ou tout type de couvre-chef, par exemple avec une bande élastique destinée à passer entre le support et le boitier, un casque audio (pour un port sur le haut du crâne), un casque de réalité virtuelle ou augmentée, un casque de chantier, de vélo, un calot de chirurgien, un masque de protection etc. Le moyen d'attache formé du support et du boîtier peut être adapté au type de vêtement ou d'accessoire auquel il est destiné à être attaché. Tout type de dispositif, languette à scratch, bouton pression, accroche mécanique ou magnétique peut être prévu pour faciliter le maintien du vêtement ou accessoire dans le moyen d'attache formé par le support flexible et le boitier.

En pratique, on pourra venir « clipser » le dispositif portatif sur le vêtement ou l'accessoire, avant qu'il ne soit porté par l'utilisateur, ou au contraire, on pourra venir « clipser » le dispositif portatif sur le vêtement ou l'accessoire une fois ce dernier positionné sur la tête de l'utilisateur.

Comme illustré sur les figures précédentes, les lamelles conductrices peuvent être avantageusement toutes sensiblement parallèles, ce qui permet de positionner le support sur le crâne par un geste de translation parallèle à la direction des lamelles conductrices, par exemple du haut de la tête vers le bas de la tête lorsqu'il s'agit d'un dispositif destiné à être porté dans une région occipitale du crâne. Pour des raisons de confort et de qualité du contact en effet, le mouvement de translation suit de préférence le sens et la direction de l'implantation des cheveux.

Les différentes branches des supports qui supportent les capteurs peuvent également être sensiblement parallèles, et parallèles à la direction des lamelles conductrices, comme cela apparaît par exemple sur les FIGS.1A - 1E et 2A - 2C. Cela présente des effets à la fois esthétiques, mécaniques et ergonomiques. En effet, les branches parallèles permettent d'appliquer correctement la pression au niveau des capteurs avec un bandeau ne recouvrant pas entièrement le support. Ainsi, le bandeau appuie sur les branches parallèles et plaque les électrodes aux extrémités. Les branches ainsi agencées parallèlement aux lamelles conductrices permettent de minimiser la partie visible du support lorsqu'il est porté et facilitent la compréhension du geste de translation nécessaire à la mise en place du dispositif.

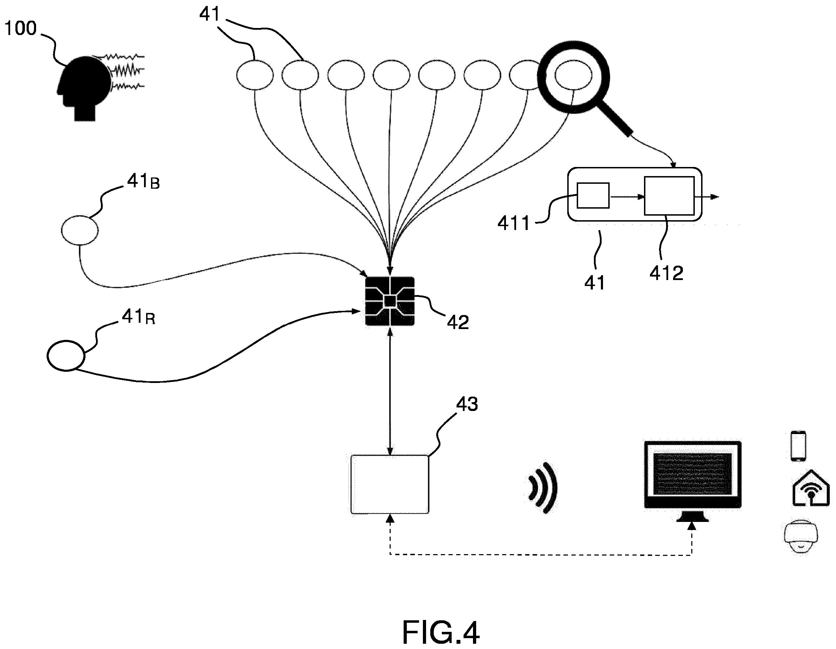

La FIG. 4 illustre un exemple d'architecture électronique pour la réception et le traitement des signaux EEG au moyen d'un dispositif portatif selon la présente description.

Comme décrit précédemment, le dispositif portatif selon la présente description comprend un nombre donné d'électrodes actives 41, par exemple entre 2 et 128, avantageusement entre 2 et 64, avantageusement entre 4 et 16. Chaque électrode active 41 comprend un capteur 411 pour la détection des signaux électriques générés par l'activité neuronale de l'utilisateur 100 et un circuit électronique de filtrage et d'amplification 412. Par exemple, chaque circuit électronique 412 comprend un filtre analogique passe-haut du premier ordre, un amplificateur et un filtre analogique passe-bas du premier ordre. Les filtres permettent de supprimer des signaux captés les composantes fréquentielles inutiles pour l'application envisagée. L'amplification permet d'adapter l'amplitude des signaux aux caractéristiques du CAN et obtenir une résolution maximale lors de la conversion. Comme illustré sur la FIG. 4, le dispositif portatif comprend également une ou plusieurs électrodes de référence 41_{R} et une électrode dite de « bias » ou de mise à la masse 41_{B}. Les électrodes actives sont connectées à un ou plusieurs convertisseurs analogique-numérique 42 (CAN), chaque CAN pouvant convertir les signaux d'un nombre donné d'électrodes actives, par exemple entre 1 et 128. L'électrode de référence est connectée à tous les convertisseurs. La ou les électrodes de référence sont positionnées de préférence en contact avec la tête de l'utilisateur dans une région éloignée de celle des autres électrodes actives ; par exemple, elles peuvent être connectées grâce à un port de connexion tel que le port 123 représenté sur la FIG. 1C et placées en contact avec l'oreille, ou maintenues par le vêtement ou accessoire auquel le dispositif portatif est attaché. Les convertisseurs 42 sont commandés par un microcontrôleur 43 et communiquent avec celui-ci par exemple par le protocole SPI (« *Serial Peripheral Interface* »). Le microprocesseur encapsule les données reçues puis les transmet vers une unité de traitement externe 44, par exemple un ordinateur, un téléphone mobile, un casque de réalité virtuelle, un système informatique automobile ou aéronautique par exemple de type ordinateur de bord de voiture ou d'avion, par exemple par la technologie Bluetooth, Wi-Fi (« *Wireless Fidelity* ») ou Li-Fi (« *Light Fidelity* »)*.* L'ensemble des composants de la chaîne d'acquisition est alimenté par une batterie (non représentée sur la FIG. 4) logée dans le boîtier.

Selon un mode de fonctionnement du dispositif portatif selon la présente description, chaque électrode active mesure une valeur de potentiel électrique à laquelle on soustrait le potentiel mesuré par l'électrode de référence (Eᵢ = Vᵢ - V_{ref}), et c'est le résultat de cette différence qui est numérisé au moyen du CAN 42 puis transmis par le microcontrôleur 43. Pour ce faire, comme illustré sur la FIG. 4, le dispositif comprend un nombre donné d'électrodes actives de mesure, par exemple entre 2 et 64, avantageusement entre 4 et 16 une ou deux électrodes actives de référence 41_{R} et le contact supplémentaire 41_{B} de mise à la masse (électrode de « bias »).

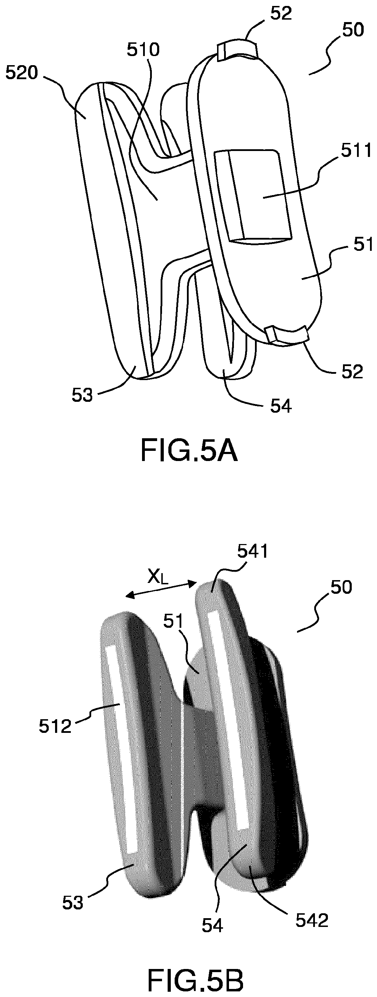

Les FIGS 5A et 5B illustrent deux vues d'un exemple de capteur selon la présente description.

Dans cet exemple, le capteur 50 comprend une base 51 destinée à être logée dans un emplacement du support flexible (non représenté) au moyen d'un dispositif de fixation 52, et plusieurs lamelles conductrices 53, 54. Les lamelles conductrices forment un contact linéique avec le scalp lorsque le dispositif est porté par l'utilisateur. Un contact linéique présente l'avantage de former une surface de contact assez grande avec le cuir chevelu, permettant une meilleure sensibilité dans la réception des signaux et un meilleur confort pour l'utilisateur. Par ailleurs, lorsque les lamelles conductrices sont agencées de façon sensiblement parallèle, les contacts linéiques ainsi formés sont compatibles avec un mouvement de translation qui écarte les cheveux lors de la mise en place du dispositif.

Les déposants ont montré qu'un nombre de 2 lamelles conductrices était optimal. Avec une seule lamelle par capteur, il peut y avoir une instabilité dans le contact formé par la lamelle sur le scalp et la répartition de la pression sur une seule lamelle peut présenter de l'inconfort pour l'utilisateur. Les déposants ont montré que 2 lamelles étaient adaptées pour couvrir une surface suffisamment faible du crâne et avoir ainsi une bonne précision de signal. L'espacement entre les lamelles répond à un compromis entre l'exigence de pouvoir laisser passer les cheveux, la mesure du signal et la répartition de la pression pour le confort de l'utilisateur. Avantageusement, les deux lamelles sont suffisamment espacées pour pouvoir laisser passer les cheveux. Par exemple, la distance bord à bord X_{L} entre les deux lamelles est supérieure à 2 mm. La distance maximale entre les lamelles dépend de la surface totale que l'on veut couvrir et du nombre de capteurs. Mais pour ne pas perdre en précision, il est préférable d'avoir une distance inférieure à 50 mm, avantageusement inférieure à 10 mm. Par exemple la distance X_{L} est comprise entre 2 et 6 mm.

Dans l'exemple des FIGS 5A et 5B, le capteur 50 comprend une structure métallique 510 intégrée à une pièce plastique 520. La structure métallique forme au niveau de la base 51 un contact 511 qui permettra d'assurer le passage du courant avec le circuit de filtrage et d'amplification et au niveau de chaque lamelle 53, 54, une zone de contact électrique 512 avec le cuir chevelu.

Au niveau du contact avec le scalp, le métal pourra être traité (plaquage argent/chlorure d'argent par exemple).

Dans l'exemple représenté que les FIGS. 5A, 5B, la lamelle au niveau de la zone de contact avec le cuir chevelu est légèrement concave, ce qui permet d'épouser plus naturellement la forme du crâne.

Comme illustré sur les FIGS 5A, 5B, les lamelles peuvent présenter une ou deux pointes (541, 542 sur la FIG. 5B), une de chaque côté du capteur pour garder la symétrie et permettre le pivot du capteur par rapport au support. La pointe du côté bas du capteur sert aussi à faciliter la pénétration du capteur dans les cheveux pour atteindre le scalp. Les pointes ne sont pas nécessairement conductrices.

Comme illustré sur les FIGS 5A, 5B, les lamelles peuvent être pleines, ce qui leur confère une meilleure rigidité.

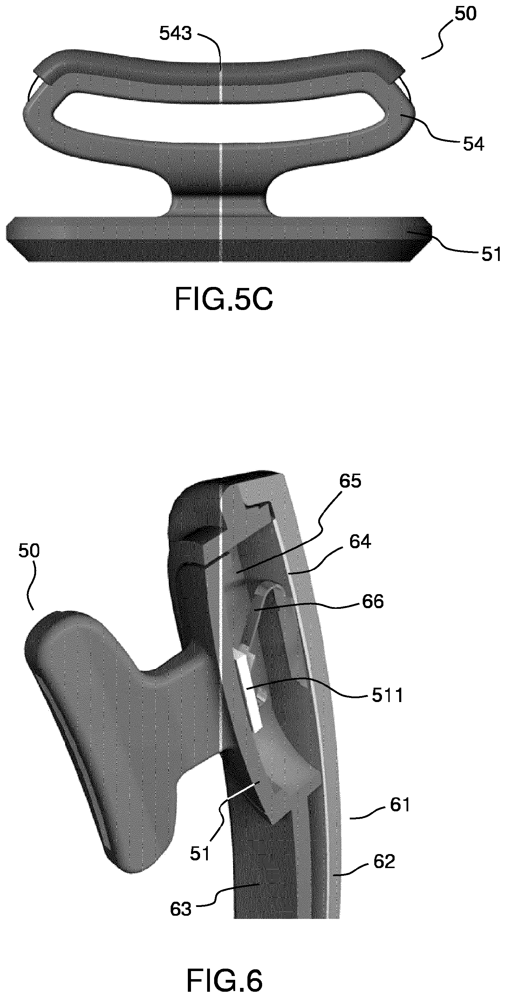

La FIG. 5C représente un autre exemple de réalisation d'un capteur selon la présente description. Dans cet exemple, un polymère conducteur, par exemple de type PEDOT:PSS, forme une couche souple 543 qui peut s'aplatir sur le scalp lorsqu'on exerce une pression et ainsi augmenter la surface de contact. La pression est alors mieux répartie sur le crâne ce qui rend le dispositif plus confortable. D'autre part, la surface de contact est plus importante, et la résistance électrique à l'interface entre le crâne et le capteur plus faible, ce qui résulte en un meilleur passage des signaux EEG dans le capteur. De plus, le polymère souple absorbe les micromouvements du dispositif par rapport au scalp de l'utilisateur et améliore la stabilité des propriétés électriques à l'interface, ce qui permet de réduire les artefacts, c'est-à-dire les variations dans les mesures, indépendantes de l'activité cérébrale. Enfin, ces polymères ont l'avantage d'être biocompatibles.

Bien entendu, d'autres formes et/ou revêtements sont possibles pour former un capteur selon la présente description. Par exemple, on pourrait se passer de la partie métallique du capteur en utilisant un matériau conducteur autre que le métal pour la structure du capteur (tel qu'un polymère conducteur).

La FIG. 6 illustre un exemple de capteur 50 tel que décrit sur les FIGS 5A, 5B monté dans un support flexible 61. Comme illustré sur la FIG. 6, la base 51 du capteur peut être montée de façon amovible dans un logement 65 du support formé entre deux plaques 62, 63 du support. Le capteur 50 est monté mobile par rapport au support flexible 61 pour permettre au dispositif de s'adapter aux différentes formes de crânes. Une lame en métal 66 avec effet ressort assure le contact électrique entre la zone conductrice 511 de la base et le circuit de filtrage et d'amplification situé sur un circuit imprimé flexible 64 interne au support flexible. La lame 66 assure grâce à un contact ponctuel avec la base du capteur une mobilité en translation sur l'axe vertical (perpendiculaire au support), et en rotation autour des 2 axes horizontaux. Ces mobilités sont contraintes par les formes du capteur et de son support.

Bien que décrite à travers un certain nombre d'exemples de réalisation détaillés, les dispositifs portatifs pour l'acquisition de signaux électroencéphalographiques selon la présente description comprennent différentes variantes, modifications et perfectionnements qui apparaîtront de façon évidente à l'homme de l'art, étant entendu que ces différentes variantes, modifications et perfectionnements font partie de la portée de l'objet de la présente description, telle que définie par les revendications qui suivent.

## Revendications

1. Dispositif (10, 20, 30) portatif pour l'acquisition de signaux électroencéphalographiques, EEG, émis par un utilisateur, le dispositif comportant:
un support (11, 21) flexible destiné à épouser une région localisée du crâne (100) de l'utilisateur;
un ensemble de capteurs (13, 411, 50) pour la détection de signaux électriques générés par l'activité neuronale de l'utilisateur, agencés sur ledit support de telle sorte à former des contacts avec le cuir chevelu lorsque le dispositif est porté par l'utilisateur;
pour chaque capteur, un circuit (412) électronique de filtrage et d'amplification des signaux électriques détectés par ledit capteur, ledit circuit électronique étant intégré dans le support flexible et formant avec ledit capteur une électrode active;
un boitier (12, 22, 32) relié mécaniquement au support flexible pour former, avec ledit support flexible, un moyen d'attache à un vêtement ou accessoire (110) destine à être porté par l'utilisateur, le boitier comportant une chaîne électronique de traitement des signaux issus desdits circuits électroniques de filtrage et d'amplification,
dans lequel chaque capteur comporte en outre une pluralité de lamelles (131, 132, 53, 54) conductrices, les lamelles conductrices formant des contacts linéiques avec le cuir chevelu lorsque le dispositif est porté par l'utilisateur, les lamelles (131, 132, 53, 54) conductrices étant déformables par pression sur le cuir chevelu de l'utilisateur.

2. Dispositif (10, 20, 30) portatif selon la revendication 1, dans lequel le support flexible est ajouré et comporte une pluralité de branches (111, 112, 113, 114, 211-215, 216, 217, 311-314, 315-318) sur lesquelles est agencée au moins une partie desdits capteurs.

3. Dispositif (10, 20, 30) portatif selon la revendication 2, dans lequel le support flexible comporte une partie (110, 310) centrale, les branches étant reliées à ladite partie centrale au moyen de branches latérales flexibles.

4. Dispositif (10, 20, 30) portatif selon l'une quelconque des revendications précédentes, dans lequel le support flexible et le boitier sont reliés mécaniquement par un point (121, 221) d'attache flexible, permettant de former un moyen d'attache en forme de clip.

5. Dispositif (10, 20, 30) portatif selon l'une quelconque des revendications précédentes, dans lequel chaque capteur est monté mobile sur ledit support flexible.

6. Dispositif (10, 20, 30) portatif selon l'une quelconque des revendications précédentes, dans lequel chaque capteur comprend une base (51) destinée à être agencée dans un logement (65) dudit support flexible, en contact électrique avec ledit circuit de filtrage et d'amplification.

7. Dispositif (10, 20, 30) portatif selon la revendication 6, dans lequel ledit contact électrique est assuré par une lame ressort (66) formant un contact ponctuel avec ladite base.

8. Dispositif (10, 20, 30) portatif selon l'une quelconque des revendications précédentes, dans lequel chaque capteur comprend deux lamelles conductrices.

9. Dispositif (10, 20, 30) portatif selon l'une quelconque des revendications précédentes, dans lequel lesdites lamelles conductrices sont agencées de façon sensiblement parallèle entre elles.

10. Dispositif (10, 20, 30) portatif selon l'une quelconque des revendications précédentes, dans lequel les lamelles conductrices comprennent un revêtement polymère conducteur formant une couche conductrice destinée à entrer en contact avec le scalp de l'utilisateur.

11. Vêtement connecté pour l'acquisition de signaux électroencéphalographiques, EEG, comportant un dispositif (10, 20, 30) portatif selon l'une quelconque des revendications précédentes.

12. Procédé d'acquisition de signaux électroencéphalographiques, EEG, émis par un utilisateur au moyen d'un dispositif (10, 20, 30) portatif selon l'une quelconque des revendications précédentes, comportant:
la mesure de signaux électriques générés par l'activité neuronale de l'utilisateur au moyen desdits capteurs (13, 411, 50) en contact avec le cuir chevelu de l'utilisateur; et
le traitement des signaux électriques issus desdits circuits (412) de filtrage et d'amplification au moyen de la chaîne électronique de traitement agencée dans ledit boitier.

## Patentansprüche

1. Tragbare Vorrichtung (10, 20, 30) zur Erfassung von von einem Benutzer emittierten Elektroenzephalographie-(EEG)-Signalen, wobei die Vorrichtung Folgendes umfasst:
eine flexible Stütze (11, 21) zum Anpassen an eine lokalisierte Region des Schädels (100) des Benutzers;
einen Satz von Sensoren (13, 411, 50) zum Erfassen von durch die neuronale Aktivität des Benutzers erzeugten elektrischen Signalen, die so auf der genannten Stütze angeordnet sind, dass sie Kontakte mit der Kopfhaut bilden, wenn die Vorrichtung vom Benutzer getragen wird;
für jeden Sensor eine elektronische Schaltung (412) zum Filtern und Verstärken der von dem genannten Sensor erfassten elektrischen Signale, wobei die genannte elektronische Schaltung in die flexible Stütze integriert ist und mit dem genannten Sensor eine aktive Elektrode bildet;
ein Gehäuse (12, 22, 32), das mechanisch mit der flexiblen Stütze verbunden ist, um mit der genannten flexiblen Stütze ein Mittel zum Anbringen an einem zum Tragen durch den Benutzer bestimmten Kleidungsstück oder Accessoire (110) zu bilden, wobei das Gehäuse eine elektronische Kette zur Verarbeitung der von den genannten elektronischen Filter- und Verstärkungsschaltungen stammenden Signale umfasst,
wobei jeder Sensor außerdem mehrere leitfähige Lamellen (131, 132, 53, 54) umfasst, wobei die leitfähigen Lamellen lineare Kontakte mit der Kopfhaut bilden, wenn die Vorrichtung vom Benutzer getragen wird, wobei die leitfähigen Lamellen (131, 132, 53, 54) durch Druck auf die Kopfhaut des Benutzers verformbar sind.

2. Tragbare Vorrichtung (10, 20, 30) nach Anspruch 1, wobei die flexible Stütze durchbrochen ist und mehrere Zweige (111, 112, 113, 114, 211-215, 216, 217, 311-314, 315-318) aufweist, auf denen mindestens ein Teil der Sensoren angeordnet ist.

3. Tragbare Vorrichtung (10, 20, 30) nach Anspruch 2, wobei die flexible Stütze einen Mittelteil (110, 310) umfasst, wobei die Zweige mittels flexibler lateraler Zweige mit dem genannten Mittelteil verbunden sind.

4. Tragbare Vorrichtung (10, 20, 30) nach einem der vorherigen Ansprüche, wobei die flexible Stütze und das Gehäuse mechanisch durch einen flexiblen Anbringungspunkt (121, 221) verbunden sind, der die Bildung eines clipförmigen Anbringungsmittels zulässt.

5. Tragbare Vorrichtung (10, 20, 30) nach einem der vorherigen Ansprüche, wobei jeder Sensor beweglich an der genannten flexiblen Stütze montiert ist.

6. Tragbare Vorrichtung (10, 20, 30) nach einem der vorherigen Ansprüche, wobei jeder Sensor eine zum Anordnen in einer Aufnahme (65) der genannten flexiblen Stütze in elektrischem Kontakt mit der genannten Filter- und Verstärkerschaltung bestimmte Basis (51) umfasst.

7. Tragbare Vorrichtung (10, 20, 30) nach Anspruch 6, wobei der genannte elektrische Kontakt durch eine Blattfeder (66) hergestellt wird, die einen Punktkontakt mit der genannten Basis bildet.

8. Tragbare Vorrichtung (10, 20, 30) nach einem der vorherigen Ansprüche, wobei jeder Sensor zwei leitfähige Lamellen umfasst.

9. Tragbare Vorrichtung (10, 20, 30) nach einem der vorherigen Ansprüche, wobei die genannten leitfähigen Lamellen im Wesentlichen parallel zueinander angeordnet sind.

10. Tragbare Vorrichtung (10, 20, 30) nach einem der vorherigen Ansprüche, wobei die leitfähigen Lamellen eine leitfähige Polymerbeschichtung umfassen, die eine leitfähige Schicht zum Inkontaktkommen mit der Kopfhaut des Benutzers bildet.

11. Angeschlossenes Kleidungsstück zur Erfassung von Elektroenzephalographie-(EEG)-Signalen, das eine tragbare Vorrichtung (10, 20, 30) nach einem der vorherigen Ansprüche umfasst.

12. Verfahren zur Erfassung von von einem Benutzer mittels einer tragbaren Vorrichtung (10, 20, 30) nach einem der vorherigen Ansprüche emittierten Elektroenzephalographie-(EEG)-Signalen, das Folgendes umfasst:
Messen von durch die neuronale Aktivität des Benutzers erzeugten elektrischen Signalen mithilfe der mit der Kopfhaut des Benutzers in Kontakt stehenden Sensoren (13, 411, 50); und
Verarbeiten der von den Filter- und Verstärkungsschaltungen (412) stammenden elektrischen Signalen mittels der in dem genannten Gehäuse angeordneten elektronischen Verarbeitungskette.

## Claims

1. A portable device (10, 20, 30) for acquiring electroencephalographic, EEG, signals emitted by a user, the device comprising:
a flexible support (11, 21) intended to fit a localized region of the skull (100) of the user;
a set of sensors (13, 411, 50) for detecting electrical signals generated by the user's neural activity, arranged on said support so as to form contacts with the scalp when the device is worn by the user;
for each sensor, an electronic circuit (412) for filtering and amplifying the electrical signals detected by said sensor, said electronic circuit being integrated into the flexible support and forming with said sensor an active electrode;
a housing (12, 22, 32) mechanically connected to the flexible support to form, with said flexible support, a means of attachment to a garment or accessory (110) intended to be worn by the user, the housing including an electronic chain for processing the signals from said electronic filtering and amplification circuits;
wherein each sensor further comprises a plurality of conductive blades (131, 132, 53, 54), the conductive blades forming linear contacts with the scalp when the device is worn by the user, the conductive blades being deformable by pressure on the scalp of the user.

2. The portable device (10, 20, 30) as claimed in claim 1, wherein the flexible support is openwork and comprises a plurality of branches (111, 112, 113, 114, 211-215, 216, 217, 311-314, 315-318) on which at least some of said sensors are arranged.

3. The portable device (10, 20, 30) as claimed in claim 2, wherein the flexible support comprises a central part (110, 310), the branches being linked to said central part by means of flexible lateral branches .

4. The portable device (10, 20, 30) as claimed in any one of the preceding claims, wherein the flexible support and the housing are linked mechanically by a flexible attachment point (121, 221), making it possible to form a clip-form attachment means.

5. The portable device (10, 20, 30) in any one of the preceding claims, wherein each sensor is movably mounted on said flexible support.

6. The portable device (10, 20, 30) as claimed in any one of the preceding claims, wherein each sensor comprises a base (51) intended to be arranged in a recess (65) of said flexible support, in electrical contact with said filtering and amplification circuit.

7. The portable device (10, 20, 30) as claimed in claim 6, wherein said electrical contact is ensured by a spring finger (66) forming a contact point with said base.

8. The portable device (10, 20, 30) as claimed in any of the preceding claims, wherein each sensor comprises two conductive blades.

9. The portable device (10, 20, 30) as claimed in any one of the preceding claims, wherein said conductive blades are arranged substantially parallel to one another.

10. The portable device (10, 20, 30) as claimed in any one of the preceding claims, wherein the conductive blades comprise a conductive polymer coating forming a conductive layer intended to enter into contact with the scalp of the user.

11. A connected garment for acquiring electroencephalographic, EEG,
signals comprising a portable device (10, 20, 30) as claimed in any one of the preceding claims.

12. A method for acquiring electroencephalographic, EEG, signals emitted by a user by means of a portable device (10, 20, 30) as claimed in any one of the preceding claims, comprising:
the measurement of electrical signals generated by the neuronal activity of the user by means of said sensors (13, 411, 50) in contact with the scalp of the user; and
the processing of the electrical signals from said filtering and amplification circuits (412) by means of the electronic processing chain arranged in said housing.
